(19) Europäisches Patentamt
European Patent Office
Office européen des brevets

(11) Veröffentlichungsnummer: **0 304 778 B1**

(12) # EUROPÄISCHE PATENTSCHRIFT

(45) Veröffentlichungstag der Patentschrift: **23.10.91**

(51) Int. Cl.⁵: **C07C 323/51**

(21) Anmeldenummer: **88113308.6**

(22) Anmeldetag: **17.08.88**

(54) **Verfahren zur Herstellung von N-acylierten Mercapto-Alpha-aminosäuren.**

(30) Priorität: **21.08.87 DE 3727897**

(43) Veröffentlichungstag der Anmeldung:
**01.03.89 Patentblatt 89/09**

(45) Bekanntmachung des Hinweises auf die Patenterteilung:
**23.10.91 Patentblatt 91/43**

(84) Benannte Vertragsstaaten:
**AT BE CH DE ES FR GB GR IT LI NL SE**

(56) Entgegenhaltungen:
**DE-A- 1 493 600**
**DE-A- 2 345 835**
**DE-B- 1 260 476**
**FR-A- 2 503 151**
**US-A- 2 496 416**

(73) Patentinhaber: **Degussa Aktiengesellschaft**
**Weissfrauenstrasse 9**
**W-6000 Frankfurt am Main 1(DE)**

(72) Erfinder: **Drauz, Karlheinz, Dr.**
**Zur Marienruhe 13**
**W-6463 Freigericht 1(DE)**
Erfinder: **Krimmer, Hans-Peter, Dr.**
**Am Weissen Turm 48**
**W-6000 Frankfurt 60(DE)**

**EP 0 304 778 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von N-acylierten Mercapto-α-aminosäuren der allgemeinen Formel

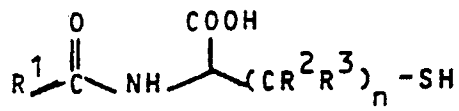

(I)

in der

R[1] Wasserstoff, ($C_1$-$C_{18}$)-Alkyl, linear oder verzweigt, unsubstituiert oder ein- bis dreifach substituiert mit Hydroxy, ($C_1$-$C_4$)-Alkoxy, Aryloxy, ($C_1$-$C_4$)-Alkylmercapto, Arylmercapto, Phosphono, ($C_1$-$C_4$)-Alkylphosphino, Arylphosphino, Carboxy, ($C_1$-$C_4$)- Alkoxycarbonyl, -($C_3$-$C_8$)-Cycloalkyl, ($C_7$-$C_{12}$)-Bicycloalkyl; Aryl-($C_1$-$C_8$)-alkyl, wobei für Aryl ein unsubstituierter oder ein- bis dreifach substituierter Phenylring steht und die Substituenten in beliebiger Stellung zueinander, voneinander unabhängig ($C_1$-$C_8$)-Alkyl linear oder verzweigt, ($C_3$-$C_8$)-Cycloalkyl, ($C_2$-$C_5$)-Alkenyl, Ethinyl, Hydroxy-($C_1$-$C_4$)-alkyl, ($C_1$-$C_4$)-Alkoxy-($C_1$-$C_4$)-alkyl, Mercapto-($C_1$-$C_4$)-alkyl, ($C_1$-$C_4$)-Alkylmercapto-($C_1$-$C_4$)-alkyl, Carboxy-($C_1$-$C_4$)-alkyl, Aryl,Pyridyl, Hydroxy, ($C_1$-$C_5$)-Alkoxy, Aryloxy, Halogen, Amino, Mono- oder Di-($C_1$-$C_5$)-alkylamino, ($C_1$-$C_4$)-Acylamino, ($C_1$-$C_4$)-Alkoxycarbonylamino, Nitro, Cyano, Phospho, Phosphono, Phosphino, ($C_1$-$C_4$)-Acyl, Carboxy, ($C_1$-$C_4$)-Alkoxycarbonyl, Mercapto, ($C_1$-$C_4$)-Alkylmercapto, Arylmercapto, Sulfo, Sulfato, Sulfano und Sulfino sein können;
Heterocyclyl-($C_1$-$C_8$)-alkyl, wobei für Heterocyclyl ein mono- oder polycyclischer, gesättigter oder ungesättigter, substituierter oder unsubstituierter heterocyclischer Rest mit einem oder mehreren Heteroatomen steht, in dem die Substituenten voneinander unabhängig die gleiche Bedeutung haben wie bei Arylalkyl definiert;
Aryl, wobei für Aryl ein oder mehrere miteinander kondensierte, substituierte oder unsubstituierte, Phenylringe stehen, in denen die Substituenten voneinander unabhängig die gleiche Bedeutung haben wie bei Arylalkyl definiert;
oder Heterocyclyl, wobei für Heterocyclyl ein mono-oder polycyclischer, gesättigter oder ungesättigter, substituierter oder unsubstituierter heterocyclischer Rest mit einem oder mehreren Heteroatomen steht, in dem die Substituenten voneinander unabhängig die gleiche Bedeutung haben wie bei Arylalkyl definiert;

$R^2$ und $R^3$ voneinander unabhängig Wasserstoff oder ($C_1$-$C_8$)-Alkyl; und

n eine ganze Zahl von 1 bis 3 bedeuten.

N-acylierte Mercapto-α-aminosäuren sind bekannt. Sie sind von Interesse als Zwischenprodukte für die Peptidsynthese, als pharmazeutische Wirkstoffe oder als kosmetische Präparationen. So ist z.B. N-Acetyl-L-cystein ein im Handel befindliches Mucolytikum. Weiterhin wird in letzter Zeit immer häufiger eine Antidotwirkung des N-Acetyl-L-cysteins gegen Paracetamolvergiftungen diskutiert. Schließlich dient N-Acetyl-L-cystein auch als kosmetisches Präparat zur permenenten Verformung des menschlichen Haares.

Die selektive N-Acylierung von Mercapto-α-aminosäuren bereitet jedoch wegen der gleichzeitigen Anwesenheit der nucleophilen Sulfhydrylgruppe neben der Aminogruppe Schwierigkeiten und ist nur unter beträchlichen Umständen möglich. Durch das erfindungsgemäße Verfahren können jedoch Mercapto-α-aminosäuren auf relativ einfache Weise selektiv an der Aminogruppe acyliert werden.

Das erfindungsgemäße Verfahren ist dadurch gekennzeichnet, daß man ein Nitril der allgemeinen Formel

$R^1$-CN (II)

in der $R^1$ eine der angegebenen Bedeutungen aufweist, mit einer Mercapto-α-aminosäure der allgemeinen Formel

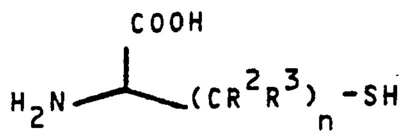

(III)

in der $R^2$ und $R^3$ eine der angegebenen Bedeutungen aufweisen, bei einer Temperatur zwischen 0° C und 100° C in Wasser oder in einer Mischung aus Wasser und einem mit Wasser zumindest teilweise mischbaren organischen Lösungsmittel in Gegenwart einer organischen oder anorganischen Base umsetzt und anschließend mit einer anorganischen oder organischen Säure die N-acylierte Mercapto-α-aminosäure der allgemeinen Formel (I) freisetzt.

Die Umsetzung zwischen dem Nitril der allgemeinen Formel (II) und der Mercapto-α-aminosäure der allgemeinen Formel (III) wird vorzugsweise bei einer Temperatur zwischen 50° C und der Siedetemperatur des Reaktionsgemisches vorgenommen.

Sofern alle Reaktionsteilnehmer wasserlöslich sind, kann die Umsetzung in reinem Wasser durchgeführt werden. Ist jedoch mindestens ein Reaktionsteilnehmer nicht ausreichend wasserlöslich, empfiehlt sich die Verwendung eines wäßrig-organischen Reaktionsmediums, also einer Mischung aus Wasser und einem mit Wasser zumindest teilweise mischbaren organischen Lösungsmittel. Als organisches Lösungsmittel können ein- oder mehrwertige lineare aliphatische, verzweigte aliphatische, cycloaliphatische, araliphatische oder duch Alkoxygruppen substituierte aliphatische Alkohole, wie Methanol, Ethanol, n-Propanol, Isopropanol, n-Butanol, Isobutanol, sec-Butanol, tert-Butanol, n-Pentanol, n-Hexanol, n-Heptanol, n-Octanol, Cyclopentanol, Cyclohexanol, Benzylalkohol, Phenylethanol, 2-Methoxyethanol, 2-Ethoxyethanol, Methoxyethoxyethanol, Ethoxyethoxyethanol, Glycol, Diethylenglycol und Glycerin; aliphatische oder cycloaliphatische mono- oder polyfunktionelle Ether, wie Diethyl-ether, Diisopropylether, Methyl-tert-butylether, Tetrahydrofuran, 1,4-Dioxan, 1,2-Dimethoxyethan, 1,2-Diethoxyethan, Diethylenglykoldimethylether und Diethylenglycoldiethylether; Carbonsäurealkylester der Ameisensäure, Essigsäure, Propionsäure, Buttersäure, Valeriansäure, Isobuttersäure und Isovaleriansäure mit Methyl-, Ethyl-, n-Propyl-, i-Propyl-, n-Butyl-, i-Butyl-, sec-Butyl-, t-Butyl-, Pentyl- und Hexylalkohol, insbesondere Essigsäureethylester; Carbonsäureamide der Ameisensäure, Essigsäure und Propionsäure mit Ammoniak, Methyl-, Dimethyl-, Ethyl-und Diethylamin, insbesondere N,N-Dimethylformamid; cycloaliphatische Lactame, wie N-Methylpyrrolidon; Trisalkylester der Phosphorsäure, wie Phosphorsäuretrimethylester und Phosphorsäuretriethylester; Tris-amide, -alkylamide und -dialkylamide der Phosphorsäure, wie Hexamethylphosphorsäuretrisamid; Dialkylsulfoxide, wie Dimethylsulfoxid, oder Dialkyl- oder Cycloalkylsulfone, wie Sulfolan; oder Harnstoffe, deren Aminogruppen mit ein, zwei, drei oder vier aliphatischen oder cycloaliphatischen Resten substituiert sein können, wie 1,3-Dimethyl-imidazolin-2-on, verwendet werden.

Das Verhältnis zwischen dem Wasser und dem organischen Lösungsmittel richtet sich jeweils nach den Gegebenheiten der zu lösenden Reaktionsteilnehmer. Es liegt bevorzugt zwischen Wasser alleine und Wasser : organisches Lösungsmittel = 1:20 (v/v). Besonders bewährte Mischungen sind solche aus Wasser und Methanol oder Isopropanol, jeweils im Verhältnis 1:3 (v/v).

Die Umsetzung zwischen dem Nitril der allgemeinen Formel (II) und der Mercapto-α-aminosäure der allgemeinen Formel (III) erfordert ferner die Gegenwart einer organischen oder anorganischen Base. Geeignete Basen sind beispielsweise Alkali-oder Erdalkalimetallhydroxide, wie Lithium-, Natrium-, Kalium-, Magnesium- und Calciumhydroxid; Alkyli- oder Erdalkalimetallcarbonate oder -hydrogencarbonate, wie Lithium-, Natrium-, Kalium-, Magnesium- und Calciumcarbonat oder -hydrogencarbonat; Amine, wie Ammoniak, Alkyl-, Dialkyl- oder Trialkylamine, z.B. Methyl-, Ethyl-, Propyl-, iso-Propyl-, n-Butyl-, iso-Butyl-, sec-Butyl-, tert-Butyl-, Dimethyl-, Diethyl-, Di-n-propyl-, Di-iso-propyl-, Di-n-butyl-, Di-iso-butyl, Di-sec-Butyl-, Trimethyl-, Triethyl-, Tri-n-propyl-, Diisopropylmethyl- und Diisopropylethylamin; oder ungesättigte organische heterocyclische Stickstoffbasen, z.B. Pyridin, α-,β-oder γ-Picolin, Lutidin, Chinolin, Isochinolin, Collidin, Pyrollidin, Piperidin, Piperazin, N-Methylpiperidin und Chinuclidin. Besonders bevorzugte Basen sind Natriumhydroxid, Kaliumcarbonat und Ammoniak. Die Base wird zweckmäßigerweise in einer solchen Menge eingesetzt, daß der pH-Wert des Reaktionsgemisches zwischen pH 6 und pH 14,vorzugsweise zwischen pH 8 und pH 10, liegt.

Es ist zweckmäßig, die Umsetzung zwischen dem Nitril der allgemeinen Formel (II) und der Mercapto-α-aminosäure der allgemeinen Formel (III) in einer Stickstoffatmosphäre vorzunehmen, um die Gefahr einer Oxidation der Mercaptoverbindungen zu den entsprechenden Disulfiden hintanzuhalten. Besonders vorteilhaft ist es, den Stickstoff während der gesamten Umsetzung durch das Reaktionsgemisch hindurchzuleiten, um gleichzeitig den entstehenden Ammoniak auszutreiben, wodurch unter Umständen die erforderliche

Reaktionszeit verkürzt wird. Je nach den eingesetzten Reaktionsteilnehmern ist eine Reaktionszeit zwischen 30 Minuten und 48 Stunden erforderlich. Im allgemeinen ist aber eine Reaktionszeit bis zu 8 Stunden ausreichend.

Während der Umsetzung entwickelt sich Ammoniak, der durch eine Gaswäsche mit Wasser adsorbiert und in einem künftigen Ansatz als Base verwendet werden kann. Nach beendeter Umsetzung muß das Reaktionsgemisch mit einer anorganischen oder organischen Säure behandelt werden, um die N-acylierte Mercapto-α-aminosäure der allgemeinen Formel (I) freizusetzen. Geeignete Säuren sind beispielsweise Halogenwasserstoffsäuren, wie Chlor-, Brom- und Jodwasserstoffsäure, Schwefelsäure, Salpetersäure, Phosphorsäure oder Kohlendioxid, sowie mono- oder polyfunktionelle organische Carbonsäuren, insbesondere Ameisensäure, Essigsäure und Propionsäure. Die Säure wird zweckmäßigerweise in einer solchen Menge eingesetzt, daß der pH-Wert des Reaktionsgemisches zwischen pH 1 und pH 7, vorzugsweise zwischen pH 2 und pH 5, liegt.

In solchen Fällen, wo ein wäßrig-organisches Reaktionsmedium eingesetzt werden muß, aber die gebildete N-acylierte Mercapto-α-aminosäure der allgemeinen Formel (I) in der Mischung gut löslich ist, ist es unter Umständen von Vorteil, vor der Zugabe der Säure das oder die Lösungsmittel, zweckmäßigerweise unter vermindertem Druck,abzudestillieren und den Rückstand in reinem Wasser aufzunehmen. Das Produkt fällt dann beim Ansäuern der wäßrigen lösung meist analysenrein aus.

In anderen Fällen ist es zur Freisetzung der gebildeten N-acylierten Mercapto-α-aminosäure der allgemeinen Formel (I) erforderlich, das Reaktionsgemisch nach Zugabe der Säure 15 Minuten bis 10 Stunden, vorzugsweise bis zu 3 Stunden, auf eine Temperatur zwischen 40° C und 100° C, insbesondere zwischen 70° C und der Siedetemperatur des Reaktionsgemisches, zu erhitzen. Beim Abkühlen wird dann meist das Produkt in reiner Form ausgeschieden.

Bei der Umsetzung von chiralen, also optisch aktiven Mercapto-α-aminosäuren der allgemeinen Formel (III) weist das erfindungsgemäße Verfahren den Vorteil auf, daß die Umsetzung unter Erhalt des Chiralitätszentrums verläuft.

Die Erfindung wird durch die nachfolgenden Beispiele näher erläutert:

Beispiel 1:

N-Nicotinoyl-D,L-cystein

41,6 g (0,4 Mol) Nicotinsäurenitril, 70,5 g (0,4 Mol) D,L-Cystein-hydrochlorid-monohydrat und 55,2 g (0,4 Mol) Kaliumcarbonat wurden in 500 ml Methanol und 500 ml Wasser 6Stunden zum Sieden erhitzt. Anschließend wurde das Lösemittel unter vermindertem Druck entfernt, der Rückstand in 300 ml Wasser aufgenommen, mit konzentrierter Salzsäure auf pH 5 eingestellt und 45 Minuten zum Sieden erhitzt. Nach dem Abkühlen konnten 78,7 g (87 % der Theorie) farbloses Produkt vom Schmelzpunkt 195° C abgesaugt werden.

| $C_9H_{10}N_2O_3S$ (226,26) | | | | | | | | |
|---|---|---|---|---|---|---|---|---|
| Berechnet: | C | 47,78 % | H | 4,45 % | H | 12,38 % | S | 14,17 % |
| Gefunden : | | 47,72 % | | 4,50 % | | 12,17 % | | 14,19 % |

$^1$H-NMR (DMSO-$d^6$) : δ = 2,64 (t; 1H, SH), 2,97 (m; 2H, β-$CH_2$), 4,49 (m; 1H, α-H), 7,55 (dd; 1H, 5'-H), 8,24 (d; 1H, 4'-H), 8,76 (d; 1H, 6'-H), 8,91 (d; 1H, NH), 9,05 (s; 1H, 2'-H) 13 (breit; 1H, COOH).

Beispiel 2:

N-Nicotinoyl-D-penicillamin -hydrochlorid

20,8 g (0,2 Mol) Nicotinsäurenitril, 29,8 g (0,2 Mol) D-Penicillamin und 13,8 g (0,1 Mol) Kaliumcarbonat wurden in 350 ml Methanol und 150 ml Wasser 6 Stunden zum Sieden erhitzt. Mit konzentrierter Salzsäure wurde auf pH 4 angesäuert und 45 Minuten zum Sieden erhitzt. Nach dem Abkühlen fielen 36,5 g (63 % der Theorie) des Produktes als Hydrochlorid vom Schmelzpunkt 165° C aus.
$[\alpha]_D^{25}$ = + 4,0° (c = 2 in 1N NaOH)

| $C_{11}H_{14}N_2O_3S \cdot HCl$ (290,77) | | | | | | | | |
|---|---|---|---|---|---|---|---|---|
| Berechnet: | C | 45,44 % | H | 5,20 % | N | 9,63 % | S | 11,03 % |
| Gefunden : | | 45,27 % | | 5,20 % | | 9,29 % | | 11,14 % |

$^1$H-NMR (DMSO-$d^6$): $\delta$ = 1,52 (2; 6H, 2 $CH_3$), 3,20 (s; 1H SH), 4,72 (d; 1H, α-H), 7,55 (dd; 1H, 5'-H), 8,26 (dt; 1H, 4'-H), 8,64 (d; 1H, NH), 8,75 (m; 1H, 6'-H), 9,05 (d; 1H, 2'-H), 12,9 (breit; 1H, COOH).

Beispiel 3:

N-(4'-Chlorbenzoyl)-D-penicillamin

27,5 g (0,2 Mol) 4-Chlorbenzonitril, 29,8 g (0,2 Mol) D-Penicillamin und 13,8 g (0,1 Mol) Kaliumcarbonat wurden in 350 ml Methanol und 150 ml Wasser 6 Stunden zum Sieden erhitzt. Nach Ansäuern mit konzentrierter Salzsäure auf pH 4 wurde eine Stunde unter Stickstoff zum Sieden erhitzt. Nach Abkühlen wurden 42,6 g (74 % der Theorie) eines farblosen Niederschlags vom Schmelzpunkt 174° C isoliert.

| $C_{12}H_{14}ClNO_3S$ ( 287,76) | | | | | | | | | | |
|---|---|---|---|---|---|---|---|---|---|---|
| Berechnet: | C | 50,09 % | H | 4,91 % | Cl | 12,32 % | N | 4,87 % | S | 11,14 % |
| Gefunden : | | 50,27 % | | 4,99 % | | 12,16 % | | 4,69 % | | 11,10 % |

$^1$H-NMR (DMSO-$d^6$):$\delta$ = 1,57 (s; 6H, 2$CH_3$), 3,20 (s; 1H, SH), 4,68 (d; 1H, α-H), 7,56 (d; 2H, Phenyl), 8,02 (d; 2H, Phenyl), 8,52 (d; 1H, NH), 13 (breit; 1H, COOH).

Beispiel 4:

N-( 4'-Brombenzoyl)-D-penicillamin

36,4 g (0,2 Mol) 4-Brombenzonitril, 29,8 g (0,2 Mol) D-Penicillamin und 13,8 g (0,1 Mol) Kaliumcarbonat wurden in 350 ml Methanol und 150 ml Wasser 6 Stunden zum Sieden erhitzt. Nach Ansäuern mit konzentrierter Salzsäure auf pH 4 wurde eine Stunde zum Sieden erhitzt. Nach dem Abkühlen wurden 53,6 g (81 % der Theorie) des Produktes als farbloser Niederschlag vom Schmelzpunkt 176° C isoliert.

| $C_{12}H_{14}BrNO_3S$(332,22) | | | | | | | | | | |
|---|---|---|---|---|---|---|---|---|---|---|
| Berechnet: | C | 43,38 % | H | 4,25 % | Br | 24,05 % | N | 4,22 % | S | 9,65 % |
| Gefunden : | | 43,27 % | | 4,44 % | | 23,91 % | | 4,36 % | | 9,19 % |

$^1$H-NMR (DMSO-$d^6$):$\delta$ = 1,57 (s; 6H, 2$CH_3$), 3,18 (s; 1H, SH) 4,67 (d; 1H, α-H), 7,70 (d; 2H, Phenyl), 7,85 (d; 2H, Phenyl), 8,52 (d; 1H, NH), 12,92 (breit; 1H, COOH).

Beispiel 5:

N-Phenacetyl-L-cystein-monohydrat

117,2 g (1 Mol) Benzylcyanid 176,2 g (1 Mol) L-Cysteinhydrochloridmonohydrat und 138 g ( 1 Mol) Kaliumcarbonat wurden in 1700 ml Methanol und 750 ml Wasser 8 Stunden unter Stickstoff zum Sieden erhitzt. Das Lösemittel wurde unter vermindertem Druck bis auf 800 ml eingeengt und der Rückstand mit konzentrierter Salzsäure auf pH 4 angesäuert. Es ergaben sich 238 g (93 % der Theorie) des Produktes vom Schmelzpunkt 105-108° C, das als Monohydrat anfiel.
$[\alpha]_D^{25}$ = -24,7° (c = 2 in 1N NaOH)

| $C_{11}H_{13}NO_3S \cdot H_2O$ (257,30) | | | | | | |
|---|---|---|---|---|---|---|
| Berechnet: | C | 51,34 % | H | 5,87 % | N | 5,44 % |
| Gefunden : | | 51,86 % | | 5,43 % | | 5,02 % |

$^1$H-NMR (DMSO-$d^6$):$\delta$ = 2,80 (m; 2H, $\beta$-$CH_2$) 3,48 (s; 1H, SH), 3,55 (s; 2H, Benzyl-$CH_2$), 4,41 (mc; 1H, $\alpha$-H), 7,30 (mc; 5H, Phenyl), 8,42 (d; 1H, NH), 13 (breit; 1H, COOH).

Beispiel 6:

N-(4-Methylphenacetyl)-D,L-cystein

26,2 g (0,2 Mol) 4-Tolylacetonitril, 35,2 g (0,2 Mol) D,L-Cysteinhydrochlorid-monohydrat und 27,6 g (0,2 Mol) Kaliumcarbonat wurden in 350 ml Methanol und 250 ml Wasser 6 Stunden zum Sieden erhitzt. Anschließend wurde das Lösemittel unter vermindertem Druck entfernt, der Rückstand in 200 ml Wasser aufgenommen und mit konzentrierter Salzsäure auf pH 4 eingestellt. Dabei fielen 40,8 g (81 % der Theorie) farbloses Produkt vom Schmelzpunkt 123° C aus.

| $C_{12}H_{15}NO_3S$ (253,32) | | | | | | | | |
|---|---|---|---|---|---|---|---|---|
| Berechnet: | C | 56,89 % | H | 5,96 % | N | 5,53 % | S | 12,67 % |
| Gefunden : | | 56,73 % | | 5,76 % | | 5,26 % | | 12,32 % |

$^1$H-NMR (DMSO-$d^6$): $\delta$ = 2,28 (s; 3H, 4′-$CH_3$), 2,37 (breites t; 1H, SH), 2,79 (m; 2H, $\beta$-$CH_2$) 3,44 (s; 2H, Benzyl-$CH_2$), 4,37 (m; 1H, $\alpha$-H), 7.13 (mc; 4H, Phenyl), 8,36 (d; 1H, NH), 12,8 (breit; 1H, COOH).

Beispiel 7:

N-(3′,5′-Dimethylphenacetyl)-D,L-cystein

29,0 g (0,2 Mol) 3,5-Dimethylbenzylcyanid, 35,2 g (0,2 Mol) D,L-Cystein-hydrochlorid-monohydrat und 27,6 g (0,2 Mol) Kaliumcarbonat wurden in 500 ml Methanol und 250 ml Wasser 6 Stunden zum Sieden erhitzt. Anschließend wurde das Lösemittel unter vermindertem Druck entfernt, der Rückstand in 200 ml Wasser aufgenommen und mit konzentrierter Salzsäure auf pH 4 eingestellt. Dabei fielen 41,8 g ( 78 % der Theorie) farbloses Produkt vom Schmelzpunkt 135° C als farblose Kristalle aus.

| $C_{13}H_{17}NO_3S$ (267,35) | | | | | | | | |
|---|---|---|---|---|---|---|---|---|
| Berechnet: | C | 58,40 % | H | 6,41 % | N | 5,24 % | S | 11,99 % |
| Gefunden : | | 58,44 % | | 6,26 % | | 5,34 % | | 12,21 % |

$^1$H-NMR (DMSO-$d^6$): $\delta$ = 2,22 (s; 6H, 3′,5′-$CH_3$), 2,39 (t; 1H, SH) 2,82 (m; 2H, $\beta$-$CH_2$), 3,46 (s; 2H, Benzyl-$CH_2$), 4,40 (m; 1H, $\alpha$ -H), 6,82 (s; 1H, 4′-H), 6,90 (s; 2H, 2′,6′-H), 8,38 (d; 1H, NH), 12,8 (breit; 1H, COOH).

Beispiel 8:

N-(4′-Fluorphenacetyl)-L-cystein-monohydrat

27,0 g (0,2 Mol) 4-Fluorbenzylcyanid, 35,2 g (0,2 Mol) L-Cysteinhydrochlorid-monohydrat und 27,6 g (0,2 Mol) Kaliumcarbonat wurden in 350 ml Methanol und 150 ml Wasser 8 Stunden zum Sieden erhitzt. Das Lösemittel wurde unter vermindertem Druck entfernt, der Rückstand in 150 ml Wasser aufgenommen und mit konzentrierter Salzsäure auf pH 4 angesäuert. Es wurden so 33,6 g (61 % der Theorie) Produkt als Hydrat vom Schmelzpunkt 115° C erhalten.
$[\alpha]_D^{25}$ = -33,8° (c = 2 in 1N NaOH).

| $C_{11}H_{12}FNO_3S \cdot H_2O$ (275,29) | | | | | | | | |
|---|---|---|---|---|---|---|---|---|
| Berechnet:<br>Gefunden : | C | 47,99 %<br>48,24 % | H | 5,13 %<br>4,95 % | N | 5,09 %<br>5,23 % | S | 11,66 %<br>11,06 % |

$^1$H-NMR (DMSO-$d^6$): $\delta$ = 2,81 (m; 2H, $\beta$-$CH_2$), 3,48 (s; 1H, SH) 3,52 (s; 2H, Benzyl-$CH_2$), 4,34 (mc; 1H, $\alpha$-H), 7,12 (mc; 2H, Phenyl), 7,31 (mc; 2H, Phenyl), 8,32 (d; 1H, NH), 12,95 (breit; 1H, COOH).

Beispiel 9:

N-(2′,4′-Dichlorphenacetyl)-D,L-cystein

37,2 g (0,2 Mol) 2,4-Dichlorbenzylcyanid, 35,2 g (0,2 Mol) D,L-Cystein-hydrochlorid-monohydrat und 15,8 g (0,2 Mol) Pyridin wurden in 500 ml 1,4-Dioxan und 300 ml Wasser 8 Stunden auf 80 °C erhitzt. Anschließend wurde das Lösemittel unter vermindertem Druck entfernt, der Rückstand in 300 ml Wasser aufgenommen und mit konzentrierter Salzsäure auf pH 3 angesäuert. Es wurden 51,1 g (83 % der Theorie) farbloses Produkt vom Schmelzpunkt 164-165 °C erhalten.

| $C_{11}H_{11}Cl_2NO_3S$ (308,18) | | | | | | | | | | |
|---|---|---|---|---|---|---|---|---|---|---|
| Berechnet:<br>Gefunden : | C | 42,87 %<br>42,50 % | H | 3,57 %<br>3,56 % | N | 4,55 %<br>4,76 % | S | 10,40 %<br>10,18 % | Cl | 23,01 %<br>23,28 % |

Beispiel 10:

N-(2′-Thienylacetyl)-D,L-cystein

24,6 g(0,2 Mol) Thiophen-2-acetonitril, 35,2 g (0,2 Mol) D,L-Cystein-hydrochlorid-monohydrat und 27,6 g (0,2 Mol) Kaliumcarbonat wurden in 500 ml Methanol und 250 ml Wasser 6 Stunden zum Sieden erhitzt. Anschließend wurde das Lösemittel unter vermindertem Druck entfernt, der Rückstand in 200 ml Wasser aufgenommen und mit konzentrierter Salzsäure auf pH 4 eingestellt. Dabei fielen 40,8 g (83 % der Theorie) farbloses Produkt vom Schmelzpunkt 138-140 °C aus.

| $C_9H_{11}NO_3S_2$ (245,32) | | | | | | | | |
|---|---|---|---|---|---|---|---|---|
| Berechnet:<br>Gefunden : | C | 44,06 %<br>44,23 % | H | 4,52 %<br>4,30 % | N | 5,71 %<br>5,61 % | S | 26,14 %<br>26,21 % |

$^1$H-NMR (DMSO-$d^6$): $\delta$ = 2,25 (breit; 1H, SH), 2,82 (m; 2H, $\beta$-$CH_2$) 3,74 (s; 2H, Benzyl-$CH_2$), 4,43 (m; 1H, $\alpha$-H), 6,96 (mc; 2H, 3′,4′-H), 7,36 (dd; 1H, 5′-H), 8,43 (d; 1H, NH) 12,9 (breit; 1H, COOH).

Beispiel 11:

N-(3′-Thienylacetyl)-D,L-cystein

24,6 g (0,2 Mol) Thiophen-3-acetonitril, 35,2 g (0,2 Mol) D,L-Cystein-hydrochlorid-monohydrat und 20,2 g (0,2 Mol) Triethylamin wurden in 150 ml Isopropanol und 150 ml Wasser 4 Stunden zum Sieden erhitzt. Anschließend wurde das Lösemittel unter vermindertem Druck entfernt, der Rückstand in 250 ml Wasser aufgenommen und mit halbkonzentrierter Schwefelsäure auf pH 4 angesäuert. Es wurden 40,8 g (83 % der Theorie) farbloses Produkt vom Schmelzpunkt 125-126 °C erhalten.

| $C_9H_{11}NO_3S_2$ ( 245,32) | | | | | | | | |
|---|---|---|---|---|---|---|---|---|
| Berechnet:<br>Gefunden : | C | 44,06 %<br>44,26 % | H | 4,52 %<br>4,31 % | N | 5,71 %<br>5,77 % | S | 26,14 %<br>26,57 % |

$^1$H-NMR (DMSO-d$^6$): $\delta$ = 2,40 (breites t; 1H, SH) 2,80 (m; 2H, $\beta$-CH$_2$), 3,52 (s; 2H, Benzyl-CH$_2$), 4,41 (m; 1H, $\alpha$-H), 7,06 (d; 1H, 4'-H), 7,28 (d; 1H, 2'-H), 7,45 (dd; 1H, 5'-H), 8,41 (d; 1H, NH), 12,9 (breit; 1H, COOH).

Beispiel 12:

N-(4'-Fluorphenacetyl)-D-penicillamin

27,0 g (0,2 Mol) 4-Fluorbenzylcyanid, 29,8 g (0,2 Mol) D-Penicillamin und 13,8 g ( 0,1 Mol) Kaliumcarbonat wurden in 350 ml Methanol und 150 ml Wasser 6 Stunden zum Sieden erhitzt. Anschließend wurde das Lösemittel unter vermindertem Druck entfernt, der Rückstand in 200 ml Wasser aufgenommen und mit konzentrierter Salzsäure auf pH 4 angesäuert. Es wurden 33,5 g (59 % der Theorie) des ausgefallenen Produktes isoliert, das nach dem Trocknen einen Schmelzpunkt von 130° C aufwies.

| $C_{13}H_{16}FNO_3S$ (285,34) | | | | | | | | |
|---|---|---|---|---|---|---|---|---|
| Berechnet:<br>Gefunden : | C | 54,72 %<br>54,79 % | H | 5,65 %<br>5,48 % | N | 4,91 %<br>5,34 % | S | 11,23 %<br>11,19 % |

$^1$H-NMR (DMSO-d$^6$): $\delta$ = 1,32 (s; 3H, CH$_3$), 1,37 (S; 3H, CH$_3$) 3,33 (breit; 1H, SH), 3,59 (dd; 2H, CH$_2$), 4,41 (d; 1H, $\alpha$-H) 7,10 (mc; 2H, Phenyl), 7,30 (mc; 2H, Phenyl), 8,30 (d; 1H, NH), 12,8 (breit; 1H, COOH).

Beispiel 13:

N-(4'-Hydroxyphenacetyl)-D-penicillamin

26,6 g (0,2 Mol) 4-Hydroxybenzylcyanid, 29,8 g (0,2 Mol) D-Penicillamin und 13,8 g (0,1 Mol) Kaliumcarbonat wurden in 350 ml Methanol und 150 ml Wasser 6 Stunden zum Sieden erhitzt. Beim Ansäuern mit konzentrierter Salzsäure auf pH 4 fielen 28,9 g ( 51 % der Theorie) Produkt vom Schmelzpunkt 157° C aus.
$[\alpha]_D^{25}$ = +13,0° (c = 2 in 1 N NaOH).
$C_{13}H_{17}NO_4S$ ( 283,34)
$^1$H-NMR (DMSO-d$^6$): $\delta$ = 1,36 (s; 3H, CH$_3$), 1,39 (s; 3H, CH$_3$), 3,42 (dd; 2H, CH$_2$), 3,60 (breit; 1H, SH), 4,40 (d; 1H, $\alpha$-H), 6,69 (d; 2H, Phenyl), 7,08 (d; 2H, Phenyl), 8,11 (d; 1H, NH), 9,40 (breit; 1H, 4'-OH), 13 (breit; 1H, COOH).

Beispiel 14:

N-(2'-Pyridoyl)-D-penicillamin

20,8 g (0,2 Mol) Picolinsäurenitril, 29,8 g (0,2 Mol) D-Penicillamin und 13,8 g (0,1 Mol) Kaliumcarbonat wurden in 350 ml Methanol und 150 ml Wasser 6 Stunden zum Sieden erhitzt. Anschließend wurde das Lösemittel unter vermindertem Druck entfernt, der Rückstand in 200 ml Wasser aufgenommen und mit konzentrierter Salzsäure auf pH 5 angesäuert. Dabei fielen 32,0 g (63 % der Theorie) farbloses Produkt vom Schmelzpunkt 150 - 152° C aus .
$C_{11}H_{14}N_2O_2S$ (254,31)
$^1$H-NMR (DMSO-d$^6$): $\delta$ = 1,40 (s; 3H, CH$_3$), 1,53 (s; 3H, CH$_3$), 3,32 (breit; 1H, SH), 4,58 (d; 1H, $\alpha$-H), 7,68 (m; 1H, Pyridin-H), 8,09 (m; 2H, Pyridin-H), 8,74 (m; 2H, Pyridin-H und NH) 13,2 (breit; 1H, COOH).

Beispiel 15:

N-Isonicotinoyl-D-penicillamin-semihydrat

20,8 g (0,2 Mol) Isonicotinsäurenitril, 29,8 g (0,2 Mol) D-Pencicillamin und 13,8 g (0,1 Mol) Kaliumcarbonat wurden in 350 ml Methanol und 150 ml Wasser 6 Stunden zum Sieden erhitzt. Das Lösemittel wurde unter vermindertem Druck entfernt, der Rückstand in 200 ml Wasser aufgenommen und mit konzentrierter Salzsäure auf pH 5 eingestellt. Dabei fielen 37,1 g (70 % der Theorie) farbloses Produkt als Semihydrat vom Schmelzpunkt 200-202 °C aus.
$[\alpha]_D^{25}$ = +2,5° (c = 2 in 1N NaOH).

| $C_{11}H_{14}N_2O_3S \cdot 1/2\ H_2O$ (263,32) | | | | | | | | |
|---|---|---|---|---|---|---|---|---|
| Berechnet:<br>Gefunden : | C | 50,18 %<br>49,37 % | H | 5,74 %<br>5,69 % | N | 10,64 %<br>10,81 % | S | 12,18 %<br>11,90 % |

$^1$H-NMR (DMSO-d$^6$): δ = 1,48 (s; 6H, 2CH$_3$), 3,22 (s; 1H, SH), 4,69 (d; 1H, α-H), 7,80 (d; 2H, Pyridin-3′, 5′-H), 8,68 (d; 1H, NH), 8,73 (d; 2H, Pyridin-2′, 6′-H), 12,9 (breit; 1H, COOH).

**Beispiel: 16**

N-Acetyl-L-cystein

12,3 g (0,3 Mol) Acetonitril wurden in 250 mℓ Wasser unter Durchleiten von Stickstoff eine Stunde lang zum Sieden erhitzt. Dann wurde auf 60 °C abgekühlt und es wurden 4,4 g (0,11 Mol) Natriumhydroxid und anschließend 12,1 g (0,1 Mol) L-Cystein zugegeben.

Das Reaktionsgemisch wurde unter leichtem Durchleiten von Stickstoff 5 Stunden lang unter Rückfluß zum Sieden erhitzt.

Anschließend wurden alle flüchtigen Bestandteile des Reaktionsgemisches unter vermindertem Druck abgedampft. Das verbleibende Öl wurde in 10 mℓ Wasser aufgenommen und mit konzentrierter Salzsäure auf pH 3 angesäuert. Beim Abkühlen auf 0 °C fiel Natriumchlorid aus, das abfiltriert wurde.

Aus dem Filtrat kristallisierten 11,5 g (71 % der Theorie) Produkt als farblose Nadeln vom Schmelzpunkt 108 - 110 °C aus.
$[\alpha]_D^{25}$ = + 4.4 (c=2; $H_2O$)

**Beispiel: 17**

N-Butanoyl-L-cystein

12,1 g (0,1 Mol) L-Cystein, 6,9 g (0,1 Mol) Butyronitril und 6,9 g (0,05 Mol) Kaliumcarbonat wurden in 150 mℓ Methanol und 80 mℓ Wasser 12 Stunden unter Durchleiten von Stickstoff zum Sieden erhitzt. Anschließend wurden alle flüchtigen Anteile des Reaktionsgemisches unter vermindertem Druck abgedampft.

Der Rückstand wurde mit konzentrierter Salzsäure auf pH 4 eingestellt und mit 100 mℓ Aceton digeriert; dabei fiel das gebildete Kaliumchlorid aus. Nach dem Absaugen wurde das Filtrat im Vakuum eingeengt, der Rückstand in 100 mℓ Wasser aufgenommen und dreimal mit je 50 mℓ Dichlormethan extrahiert, um überschüssige organische Bestandteile zu entfernen. Die wässrige Phase wurde unter vermindertem Druck eingeengt. Man erhält 11,5 g (60 % d. Theorie) Produkt als hellgelbes Öl.

Beim Versuch das Öl durch Destillation zu reinigen, trat Zersetzung ein. Auch Versuche zur Kristallisation brachten keinen Erfolg.

F. Nome und J. Feuchler beschreiben in J. Am. Chem. Soc. 99, 1554 (1977) ebenfalls N-Butanoyl-L-cystein als Öl, ohne irgenwelche physikalischen Daten anzugeben. Das Produkt wird in dieser Literaturstelle weiter zu Folgeprodukten umgesetzt.

Zur Charakterisierung wurde deshalb das Öl in 20 mℓ Isopropanol aufgenommen und gasförmiger Ammoniak eingeleitet. Dabei fiel das N-Butanoyl-L-cystein-ammoniumsalz als farblose Kristalle vom Schmelzpunkt 136 - 139 °C aus
$[\alpha]_D^{20}$ = + 2,9 (c = 5, $H_2O$)

| $C_7H_{16}N_2O_3S$ (208,21) | | | | | | | | |
|---|---|---|---|---|---|---|---|---|
| Berechnet: | C | 40,38 % | H | 7,75 % | N | 13,45 % | S | 15,40 % |
| Gefunden : | | 40,19 % | | 7,61 % | | 13,80 % | | 15,07 % |

$^{1}H$-NMR (DMSO-$d^6$): $\delta$ = 0,85 (t; 3 H, $CH_3$), 1,50 (sextett; 2 H, $CH_3$-$CH_2$), 2,11 (t; 2 H, $CH_2$-CO), 2,81 (ddd; 2 H, $CH_2$-S), 4,04 (mc; 1 H, CH), 5,6 - 7,2 (breit; 5 H, $NH_4^+$ und SH), 7,42 (d; 1 H, NH).

**Beispiel: 18**

N-[3'-(Methoxycarbonyl)-propanoyl]-L-cystein

6,7 g (0,059 Mol) 3-Cyano-propionsäuremethylester, 7,2 g (0,059 Mol) L-Cystein und 5,4 g (0,08 Mol) 25 %iger wässriger Ammoniak wurden in 90 mℓ Methanol und 45 mℓ Wasser unter Durchleiten von Stickstoff 4 Stunden zum Sieden erhitzt. Danach wurden alle flüchtigen Anteile des Reaktionsgemisches unter vermindertem Druck abgedampft. Der ölige Rückstand wurde mit konzentrierter Salzsäure auf pH 4 angesäuert und anschließend mit 100 mℓ Aceton digeriert, wobei Ammoniumchlorid ausgefällt wird. Nach dem Abfiltrieren wurde das Filtrat im Vakuum eingeengt und der Rückstand in 100 mℓ Wasser aufgenommen.

Es wurde dreimal mit je 50 mℓ Dichlormethan extraliert um überschüssige organische Bestandteile zu entfernen. Die wässrige Phase wurde unter vermindertem Druck eingeengt. Man erhielt 11,5 g (83 % der Theorie) Produkt als farbloses Öl.

Versuche zur Destillation führten zur Zersetzung.

$^{1}H$-NMR (DMSO-$d^6$): $\delta$ = 2,25 - 2,50 (m; 4 H, $CH_2$-$CH_2$), 2,79 (ddd; 2 H, $CH_2$-S) 3,57 (s; 3 H, $COOCH_3$), 4,08 (mc; 1 H, CH), 7,62 (d; 1 H, NH), 10,8 (breit; 1 H, COOH).

**Patentansprüche**

1. Verfahren zur Herstellung von N-acylierten Mercapto-$\alpha$-aminosäuren der allgemeinen Formel

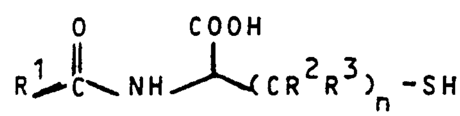

(I)

in der

$R^1$ Wasserstoff, ($C_1$-$C_{18}$)-Alkyl, linear oder verzweigt, unsubstituiert oder ein- bis dreifach substituiert mit Hydroxy, ($C_1$-$C_4$)-Alkoxy, Aryloxy, ($C_1$-$C_4$-)-Alkylmercapto, Arylmercapto, Phosphono, ($C_1$-$C_4$)-Alkylphosphino, Arylphosphino, Carboxy, ($C_1$-$C_4$)-Alkoxycarbonyl, ($C_3$-$C_8$)-Cycloalkyl, ($C_7$-$C_{12}$)-Bicycloalkyl;

Aryl-($C_1$-$C_8$)-alkyl, wobei für Aryl ein unsubstituierter oder ein- bis dreifach substituierter Phenylring steht und die Substituenten in beliebiger Stellung zueinander, voneinander unabhängig ($C_1$-$C_8$)-Alkyl linear oder verzweigt, ($C_3$-$C_8$)-Cycloalkyl, ($C_2$-$C_5$)-Alkenyl, Ethinyl, Hydroxy-($C_1$-$C_4$)-alkyl, ($C_1$-$C_4$)-Alkoxy-($C_1$-$C_4$)-alkyl, Mercapto-($C_1$-$C_4$)-alkyl, ($C_1$-$C_4$)-Alkylmercapto-($C_1$-$C_4$)-alkyl, Carboxy-($C_1$-$C_4$)-alkyl, Aryl,Pyridyl, Hydroxy, ($C_1$-$C_5$)-Alkoxy, Aryloxy, Halogen, Amino, Mono- oder Di-($C_1$-$C_5$)-alkylamino, ($C_1$-$C_4$)-Acylamino, ($C_1$-$C_4$)-Alkoxycarbonylamino, Nitro, Cyano, Phospho, Phosphono, Phosphino, ($C_1$-$C_4$)-Acyl, Carboxy, ($C_1$-$C_4$)-Alkoxycarbonyl, Mercapto, ($C_1$-$C_4$)-Alkylmercapto, Arylmercapto, Sulfo, Sulfato, Sulfano und Sulfino sein können;

Heterocyclyl-($C_1$-$C_8$)-alkyl, wobei für Heterocyclyl ein mono- oder polycyclischer, gesättigter oder ungesättigter, substituierter oder unsubstituierter heterocyclischer Rest mit einem oder mehreren Heteroatomen steht, in dem die Substituenten voneinander unabhängig die gleiche Bedeutung haben wie bei Arylalkyl definiert;

Aryl, wobei für Aryl ein oder mehrere miteinander kondensierte, substituierte oder unsubstituierte, Phenylringe stehen, in denen die Substituenten voneinander unabhängig die gleiche Bedeutung haben wie bei Arylalkyl definiert;

oder Heterocyclyl, wobei für Heterocyclyl ein mono-oder polycyclischer, gesättigter oder ungesättigter, substituierter oder unsubstituierter heterocyclischer Rest mit einem oder mehreren Heteroatomen steht, in dem die Substituenten voneinander unabhängig die gleiche Bedeutung haben wie bei Arylalkyl definiert;

$R^2$ und $R^3$ voneinander unabhängig Wasserstoff oder ($C_1$-$C_8$)-Alkyl; und

n eine ganze Zahl von 1 bis 3 bedeuten,

dadurch gekennzeichnet, daß man ein Nitril der allgemeinen Formel

$R^1$-CN (II)

in der $R^1$ eine der angegebenen Bedeutungen aufweist, mit einer Mercapto-α-aminosäure der allgemeinen Formel

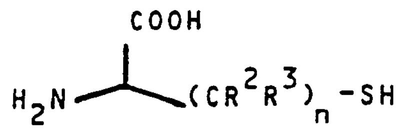

(III)

in der $R^2$ und $R^3$ eine der angegebenen Bedeutungen aufweisen, bei einer Temperatur zwischen 0 °C und 100 °C in Wasser oder in einer Mischung aus Wasser und einem mit Wasser zumindest teilweise mischbaren organischen Lösungsmittel in Gegenwart einer organischen oder anorganischen Base umsetzt und anschließend mit einer anorganischen oder organischen Säure die N-acylierte Mercapto-α-aminosäure der allgemeinen Formel (I) freisetzt.

2. Verfahren nach Anspruch 1, dadurch gekennzeichnet, daß man die Base in einer solchen Menge zusetzt, daß der pH-Wert des Reaktionsgemisches zwischen pH 6 und pH 14 liegt.

3. Verfahren nach Anspruch 1 oder 2 dadurch gekennzeichnet, daß man die Umsetzung in einer Stickstoffatmosphäre vornimmt.

4. Verfahren nach Anspruch 3, dadurch gekennzeichnet, daß man den Stickstoff während der Umsetzung durch das Reaktionsgemisch hindurchleitet.

5. Verfahren nach einem der Ansprüche 1 bis 4, dadurch gekennzeichnet, daß man vor Zugabe der Säure das oder die Lösungsmittel abdestilliert und den Rückstand in Wasser aufnimmt.

6. Verfahren nach einem der Ansprüche 1 bis 5, dadurch gekennzeichnet, daß man das Reaktionsgemisch nach Zugabe der Säure auf eine Temperatur zwischen 40 °C und 100 °C erhitzt.

7. Verfahren nach einem der Ansprüche 1 bis 6, dadurch gekennzeichnet, daß man die Säure in einer solchen Menge zusetzt, daß der pH-Wert des Reaktionsgemisches zwischen pH 1 und pH 7 liegt.

## Claims

1. A process for the production of N-acylated mercaptoα-aminoacids corresponding to the following general formula

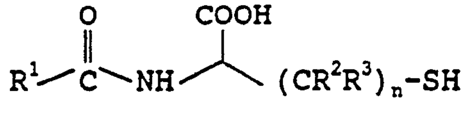

(I)

in which

$R^1$ represents hydrogen, ($C_{1-18}$) alkyl, linear or branched, unsubstituted or substituted 1

to 3 times by hydroxy, $(c_{1-4})$ alkoxy, aryloxy, $(C_{1-4})$ alkyl mercapto, aryl mercapto, phosphono, $(c_{1-4})$ alkylphosphino, arylphosphino, carboxy, $(c_{1-4})$ alkoxycarbonyl, $(C_{3-8})$ cycloalkyl, $(C_{7-12})$ bicycloalkyl;
aryl $(c_{1-8})$ alkyl, aryl being an unsubstituted or monoto tri-substituted phenyl ring and the substituents in any position to one another and independently of one another may be $(C_{1-8})$ alkyl, linear or branched, $(C_{3-8})$ cycloalkyl, $(C_{2-5})$ alkenyl, ethynyl, hydroxy$(C_{1-4})$-alkyl, $(C_{1-4})$-alkoxy- $(C_{1-4})$-alkyl, mercapto-$(C_{1-4})$-alkyl, $(C_{1-4})$-alkylmercapto- $(C_{1-4})$-alkyl, carboxy-$(C_{1-4})$-alkyl, aryl, pyridyl, hydroxy, $(C_{1-5})$ alkoxy, aryloxy, halogen, amino, mono- or di-$(C_{1-5})$-alkylamino, $(C_{1-4})$ acylamino, $C_{1-4})$ alkoxycarbonylamino, nitro, cyano, phospho, phosphono, phosphino, $(C_{1-4})$ acyl, carboxy, $(C_{1-4})$ alkoxycarbonyl, mercapto, $C_{1-4})$ alkylmercapto, arylmercapto, sulfo, sulfato, sulfano and sulfino; heterocyclyl-$(C_{1-8})$-alkyl, heterocyclyl being a monoor polycyclic, saturated or unsaturated, substituted or unsubstituted heterocyclic radical containing one or more hetero atoms, in which the substituents independently of one another have the same meanings as defined for arylalkyl;
aryl, aryl representing one or more substituted or unsubstituted phenyl rings fused to one another, in which the substituents independently of one another have the same meanings as defined for arylalkyl;
or heterocyclyl, heterocyclyl representing a monocyclic or polycyclic, saturated or unsaturated, substituted or unsubstituted heterocyclic radical containing one or more heteroatoms, in which the substituents independently of one another have the same meanings as defined for arylalkyl;

$R^2$ and $R^3$ independently of one another represent hydrogen or $(c_{1-8})$ alkyl; and
n is an integer of 1 to 3,

characterized in that a nitrile corresponding to the following general formula

$R^1$-CN (II)

in which $R^1$ has one of the meanings defined above, is reacted with a mercapto-α-aminoacid corresponding to the following general formula

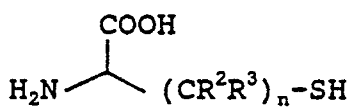

(III)

in which $R^2$ and $R^3$ have one of the meanings defined above, at a temperature of 0°C to 100°C in water or in a mixture of water and an at least partly water-miscible organic solvent in the presence of an organic or inorganic base and the N-acylated mercapto-α-aminoacid corresponding to general formula (I) is subsequently released with an inorganic or organic acid.

2. A process as claimed in claim 1, characterized in that the base is added in such a quantity that the pH value of the reaction mixture is between pH 6 and pH 14.

3. A process as claimed in claim 1 or 2, characterized in that the reaction is carried out in a nitrogen atmosphere.

4. A process as claimed in claim 3, characterized in that nitrogen is passed through the reaction mixture during the reaction.

5. A process as claimed in any of claims 1 to 4, characterized in that, before addition of the acid, the solvent(s) is/are distilled off and the residue is taken up in water.

6. A process as claimed in any of claims 1 to 5, characterized in that, after addition of the acid, the reaction mixture is heated to a temperature of 40°C to 100°C.

7. A process as claimed in any of claims 1 to 6, characterized in that the acid is added in such a quantity

that the pH value of the reaction mixture is between pH 1 and pH 7.

**Revendications**

**1.** Procédé pour la préparation de mercapto-α-aminoacides N-acylés de formule générale :

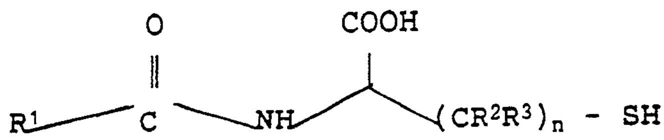

(I)

dans laquelle :

$R^1$ représente un atome d'hydrogène, un radical alkyle en ($C_1$-$C_{18}$)- linéaire ou ramidifé, non sutitué ou substitué de une à trois fois par un ou des groupes hydroxy, alcoxy en ($C_1$-$C_4$)- aryloxy, alkyl ($C_1$-$C_4$-)-mercapto, arylmercapto, phosphono, alkyl ($C_1$-$C_4$)-phosphino, arylphosphino, carboxy, alcoxy ($C_1$-$C_4$)-carboxyle, cycloalkyle en ($C_3$-$C_8$)-bicycloalkyle en ($C_7$-$c_{12}$); un radical aryl-alkyle ($C_1$-$C_8$), le fragment aryle étant représenté par un noyau phényle non substitué ou substitué de une à trois fois et les substituants pouvant être, en position quelconque les uns par rapport aux autres, indépendamment les uns des autres, des groupes alkyle en ($c_1$-$c_8$)- linéaires ou ramifiés, cycloalkyle en ($C_3$-$C_8$)- alcényle en ($C_2$-$C_5$)-éthinyle, hydroxyalkyle - ($C_1$-$C_4$)-alcoxy-($C_1$-$C_4$)-alkyle- ($C_1$-$C_4$)- mercaptoalkyle-($C_1$-$C_4$), alkyle($C_1$-$C_4$)- mercaptoalkyle - ($C_1$-$C_4$) - carboxyalkyle-($C_1$-$C_4$)- aryle, pyridyle, hydroxy, alcoxy-($C_1$-$C_5$)-aryloxy, des atomes d'halogène, un ou des groupes amino, mono- ou dialkyl - ($C_1$-$C_5$)-amino, acyl-($C_1$-$C_4$)-amino, alcoxy-($C_1$-$C_4$) carboxylamino, nitro, cyano, phospho, phosphono, phosphino, acyle en ($C_1$-$C_4$), carboxy, alcoxy ($C_1$-$C_4$)-carbonyle, mercapto, alkyl-($C_1$-$C_4$)-mercapto, arylmercapto, sulfo, sulfato, sulfano et sulfino;
un radical éthérocycloalkyle ($C_1$-$C_8$), "hétérocyclo" représentant un reste hétérocyclique monoou polycyclique saturé ou non saturé, substitué ou non substitué, comportant un ou plusieurs hétéroatomes, dans lequel les substituants ont, indépendamment les uns des autres, les mêmes significations que celles définies dans le cas du radical arylalkyle,
un radical aryle, "aryle" représentant un ou plusieurs noyaux phényle condensés les uns avec les autres, substitués ou non substitués, dans lesquels les substituants ont, indépendamment les uns des autres, les mêmes significations que celles définies dans le cas du radical arylalkyle, ou un radical hétérocyclique, le radical hétérocyclique étant représenté par un radical hétérocyclique mono- ou polycyclique saturé ou non saturé, substitué ou non substitué, comportant un ou plusieurs hétéroatomes, dans lequel les substituants ont, indépendamment les uns des autres, les mêmes significations que celles définies dans le cas du radical arylalkyle;

$R^2$ et $R^3$ représentent, indépendamment l'un de l'autre, un atome d'hydrogène ou un groupe alkyle en ($C_1$-$C_8$); et

n représente un nombre entier allant de 1 à 3,

caractérisé en ce que l'on fait réagir un nitrile de formule générale

$R^1$ - CN (II)

dans laquelle $R^1$ a les significations indiquées, avec un mercapto-α-aminoacide de formule générale

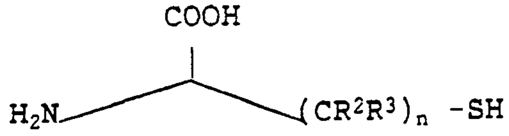

(III)

dans laquelle
$R^2$ et $R^3$ ont les significations indiquées, à une température comprise entre 0°C et 100°C, dans de

l'eau ou dans un mélange d'eau et d'un solvant organique au moins partiellement miscible à l'eau, en présence d'une base organique ou minérale, et ensuite on libère à l'aide d'un acide organique ou minéral le mercapto-α-aminoacide-N-acylé de formule générale (I).

**2.** Procédé selon la revendication 1, caractérisé en ce que l'on ajoute la base en une quantité telle que le pH du mélange réactionnel est compris entre 6 et 14.

**3.** Procédé selon la revendication 1 ou 2, caractérisé en ce que l'on effectue la réaction dans une atmosphère d'azote.

**4.** Procédé selon la revendication 3, caractérisé en ce que, pendant la réaction, on fait passer l'azote à travers le mélange réactionnel.

**5.** Procédé selon l'une quelconque des revendications 1 à 4, caractérisé en ce que, avant l'addition de l'acide, on élimine le solvant par distillation et on reprend le résidu dans de l'eau.

**6.** Procédé selon l'une quelconque des revendications 1 à 5, caractérisé en ce que, après addition de l'acide, on chauffe le mélange réactionnel à une température comprise entre 40 et 100°C.

**7.** Procédé selon l'une quelconque des revendications 1 à 6, caractérisé en ce que l'on ajoute l'acide en une quantité telle que le pH du mélange réactionnel se situe entre 1 et 7.